(19) Europäisches Patentamt
European Patent Office
Office européen des brevets

(11) Publication number: 0 350 921
A1

(12) EUROPEAN PATENT APPLICATION

(21) Application number: 89112856.3

(22) Date of filing: 13.07.89

(51) Int. Cl.4: C07C 45/50 , C07C 29/16 , C07C 27/22

(30) Priority: 14.07.88 US 218895

(43) Date of publication of application: 17.01.90 Bulletin 90/03

(84) Designated Contracting States: BE DE ES FR GB IT NL SE

(71) Applicant: UNION CARBIDE CHEMICALS AND PLASTICS COMPANY, INC.
39 Old Ridgebury Road
Danbury Connecticut 06817-0001(US)

(72) Inventor: Abatjoglou, Anthony George
1504, Lyndale Drive
Charleston West Virginia 25314(US)
Inventor: Bryant, David Robert
1201, Shady Way
South Charleston West Virginia 25309(US)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

# (54) Process for producing alcohols and aldehydes.

(57) An improved cobalt catalyzed oxo reaction process for producing alcohols and aldehydes which comprises employing a monosulfonated tertiary metal salt ligand and phase separating the alcohol and aldehyde reaction products from the oxo reaction product composition by the use of added water or both added water and an added non-polar hydrocarbon compound.

EP 0 350 921 A1

# PROCESS FOR PRODUCING ALCOHOLS AND ALDEHYDES

This invention relates to an improved process for the production of alcohols and aldehydes by the oxo reaction of an olefinic compound with carbon monoxide and hydrogen in the presence of an organic solubilized cobalt-phosphorus ligand complex catalyst, the improvement which comprises employing as said complex catalyst a cobalt-monosulfonated tertiary phosphine metal salt ligand complex catalyst and separating and recovering the alcohol and aldehyde product from the oxo reaction product composition of said process by phase separation.

The production oxo reaction product compositions of alcohols and aldehydes wherein the process is catalyzed with a cobalt-phosphorus ligand complex catalyst is well known in the art. However such processes have heretofore in general suffered from certain drawbacks particularly when the oxo reaction is applied to high molecular weight olefins, e.g., insufficient catalytic stability and/or activity and the difficulty encountered in effectively separating the high boiling alcohol and aldehyde products from the cobalt-phosphorus ligand complex containing reaction product composition by means of distillation. U.S. Patent 3,959,385 proposes to overcome such drawbacks by the use of certain carboxylated tertiary phosphine ligand salts. However said USP 3,959,385 advocates only distillation as a means for separating its oxo reaction products from the catalyst also contained in the product composition. However, distillation of high molecular weight alcohols and aldehydes (e.g., having from 7 to 31 carbon atoms) will also obviously lead to a high loss of the oxo reaction solvents advocated by U.S.P. 3,959,385, as a result of being distilled along with the oxo products, thus rendering any continuous catalyst recycle oxo process operation highly impractical.

Therefore there remains a need in the oxo process art for a highly stable and active cobalt-phosphorus ligand catalyst and a more effective and simple method for efficiently separating high molecular weight alcohol and aldehyde products from such oxo reaction product compositions, and which might serve as a basis for a practical continuous catalyst recycle oxo process.

It is now considered that the oxo production of high molecular weight (e.g., $C_7$ to $C_{31}$) alcohol and aldehyde products may be improved by the use of cobalt-monosulfonated tertiary phosphine metal salt ligand complex catalysts followed by the separation and recovery of such alcohol and aldehyde products from their oxo reaction product compositions by means of phase separation brought on by treating said compositions with added water, or both added water and an added non-polar hydrocarbon compound. It is submitted that such an improved oxo process can lead to an overall phase separation or partitioning of all the main components of such oxo reaction product compositions that is superior to heretofore conventional oxo processes that advocate and rely upon distillation as means for such separation of such alcohol and aldehyde products. For instance, oxo reaction product compositions, produced by the improved oxo reaction process of this invention, upon having been mixed with added water, or both added water and an added non-polar hydrocarbon compound can rapidly form two liquid phases, a non-polar phase consisting essentially of the alcohol and aldehyde products and whatever other non-polar compounds that might be present in the oxo reaction product composition so treated (e.g., the added non-polar hydrocarbon compound when used) and a polar phase consisting essentially of the cobalt-monosulfonated tertiary phosphine metal salt ligand complex and whatever other polar compounds that might be present in the oxo reaction product composition so treated (e.g., free monosulfonated tertiary phosphine metal salt ligand, an organic solubilizing agent for said complex and said free ligand, and added water). The alcohol and aldehyde product containing non-polar phase (which is normally the top phase) may then be easily separated and recovered from the cobalt-monosulfonated tertiary phosphine metal salt ligand complex containing polar phase (normally the bottom phase) by any conventional method desired, e.g., by simple decantation. Moreover said polar phase, after removal of the water, can be recyled to the oxo reactor if desired so that the oxo reaction may be carried out in a continuous catalyst recycle oxo process operation.

Thus it is an object of this invention to provide an improved oxo reaction process for producing and separating high molecular weight alcohol and aldehyde products from their oxo reaction product compositions, said improvement also serving as the basis for a continuous catalyst recycle oxo process. Other objects and advantages of this invention will become readily apparent from the following written description and appended claims.

Accordingly, a generic aspect of this invention can be described as an improved process for the production of alcohols and aldehydes by an oxo process comprising reacting an olefinic compound containing from 6 to 30 carbon atoms with carbon monoxide and hydrogen in the presence of a solubilized cobalt-phosphorus ligand complex, solubilized free phosphorus ligand and an organic solvent for said complex catalyst and said free ligand, the improvement which comprises employing, as the phosphorus

ligand of said complex catalyst and as said free phosphorus ligand, a monosulfonated tertiary phosphine lig and having the formula:

(I) 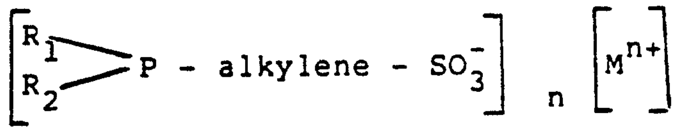

wherein $R_1$ and $R_2$ each individually represent a radical containing from 1 to 30 carbon atoms selected f rom the class consisting of alkyl and cycloalkyl radicals, wherein the divalent alkylene radical contains from 2 to 12 carbon atoms, wherein M represents a metal cation selected from the group consisting of alkali and alkaline earth metals, and wherein n has a value of 1 or 2 corresponding to the valance of the particular metal cation represented by M; and employing as the organic solvent for said complex catalyst and said free ligand a polar organic solvent; and (1) phase separating the alcohol and aldehyde products from the oxo reaction product composition of said process by treating said composition with added water, or both added water and an added non-polar hydrocarbon to form an alcohol and aldehyde product containing non-polar phase and a cobalt-monosulfonated tertiary phosphine metal salt ligand complex containing polar phase, and (2) recovering said non-polar phase from said polar phase.

As noted above, the subject invention is directed to an improved cobalt catalyzed oxo reaction process for producing alcohols and aldehydes and such oxo processes are well known in the art. Accordingly the reaction conditions for effecting such oxo reactions are not narrowly critical and may be include any such conditions heretofore conventionally advocated and/or employed. For example in general such oxo processes may involve the reaction of an olefinically unsaturated compound with carbon monoxide and hydrogen at high temperatures and pressures in the presence of a solubilized cobalt-phosphorus ligand complex catalyst, solubilized free phosphorus ligand and an organic solvent for said complex catalyst and said free ligand. Preferably the oxo process of this invention is one in which the ratio of alcohols to aldehydes in the oxo reaction product composition is at least 3 to 1.

Since the subject invention is considered to be particularly suitable for producing high molecular weight alcohol and aldehyde products containing from 7 to 31 carbon atoms, the olefinic compound starting materials of this invention are those containing from 6 to 30 carbon atoms. Such compounds can be terminally or internally unsaturated and be of straight chain, branched chain or cyclic structures, as well as be olefin mixtures, such as obtained from the oligomerization of propene, butene, isobutene, etc., such as so called dimeric, trimeric or tetrameric propylene, codibutylene, and the like, as disclosed e.g. in U.S. Patents 4,518,809 and 4,528,403, and may further contain one or more ethylenic unsaturated groups. Of course mixtures of two or more different olefinic compounds may be employed as the starting material if desired, and such olefinic compounds may also contain one or more groups or substituents which do not unduly adversely interfere with the oxo reaction process or phase separation procedure of this invention. More preferably such olefinic compounds are those containing from 6 to 16 carbon atoms, and mixtures thereof. Illustrative olefinic compounds include, e.g, 1-hexene, 1-heptene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, 1-docosene, 1-tetracosene, 1-hexacosene, 1-octacosene, 1-triacontene, 2-octene, styrene, dimeric propylene, trimeric propylene, tetrameric propylene, codibutylene, 3-phenyl-1-propene, 1,4-hexadiene, 1,7-octadiene, 3-cyclohexyl-1-butene, hex-1-en-4-ol, oct-1-en-4-ol, allyl propionate, allyl butyrate, n-propyl-7-octenoate, 4-methyl styrene, 4-isopropyl styrene, 4-tert-butyl styrene, alpha-methyl styrene, 4-tert-butyl-alpha-methyl styrene, 1,3-diisopropenyl-benzene, eugenol, iso-eugenol, safrole, iso-safrole, anethol, 4-allylanisole, indene, limonene, beta-pinene, dicyclopentadiene, cyclooctadiene, camphene, linalool, and the like.

The carbon monoxide and hydrogen gases employed are often referred to as syn gas and may be employed in $H_2$:CO molar ratios ranging from about 1:10 to 100:1 or higher, the more preferred hydrogen to carbon monoxide molar ratios being from about 1 to 1 to about 10.1 and in general ratios from about 1:1 to about 3:1 should be suitable for most purposes.

In general the oxo reaction may be carried out at a reaction temperature of from about 160 °C to about 275 °C, while reaction temperatures in the range of from about 175 °C to about 225 °C may be more preferred. In addition the oxo reaction is also carried out under pressure wherein the total pressure of $H_2$ and CO may range from about 600 psig. to about 6000 psig., while such a total pressure in the range of from about 900 psig. to about 2600 psig. may be more preferred. Illustrative partial pressures of hydrogen gas include those in the range of from about 300 psig. to about 4000 psig. and preferably from about 600

psig. to about 1600 psig. Illustrative partial pressures of carbon monoxide include those in the range of from about 300 psig. to about 2000 psig. and preferably from about 300 psig. to about 1000 psig.

The improved oxo reaction process of this invention is carried out in the presence of a polar organic solvent for the cobalt-phosphorus ligand complex catalyst and the free phosphorus ligand also present in the oxo reaction medium. Illustrative polar organic solubilizing agents are those polar organic liquids having a molecular weight having a molecular weight of less than 250 and a Hildebrand solubility value of 10 or higher, and mixtures thereof. Examples of such polar compounds (along with their Hildebrand solubility parameters) include lower alcohols e.g., methanol (12.9), ethanol (11.2), propanol (10.2), isopropanol (10.2) and the like; as well as, nitriles e.g., benzonitrile (10.7), acetonitrile (11.8), propionitrile, and the like; amides e.g., dimethylformamide (11.5), dimethylacetamide, N-methyl pyrrolidone (14.8), N-methyl piperidone, 1,5-dimethyl pyrrolidone,2-pyrrolidone, 2-hydroxyethyl pyrrolidone, N-dodecyl pyrrolidone, N-ethyl pyrrolidone, N-cyclohexyl pyrrolidone, 1,2-di (pyrrolidone) ethane, N. N-dimethylpropionamide, and the like: glycols e.g., ethylene glycol, propylene glycol and the like; polyglycols e.g., diethylene glycol, triethylene glycol, tetraethylene glycol, dipropylene glycol, tripropylene glycol, and the like; sulfoxides e.g., dimethyl sulfoxide (12.8) and the like; sulfones e.g., dimethyl sulfone, sulfolane, and the like; and the like. Hildebrand solubility values are an empirical measure of the relative polarity of an organic compound and are described, e.g., in "Introduction to Modern Liquid Chromatography" by L. R. Snyder and J. J. Kirkland, pp. 215-218 (1974) a Wiley-Interscience publication, (John Wiley & Sons) and "The Solubility of Non-Electrolytes", J. H. Hildebrand and R. L. Scott, pp. 424-434, Dover Publications Inc., New York (1964).

Of course, it is to be understood that such polar organic solubilizing liquids may be employed individually or as mixtures of two or more different polar organic liquid compounds and regardless of whether or not such compounds are employed individually or as mixtures. The amount of such added polar organic solubilizing agents present in the oxo reaction product medium of a given oxo reaction process need only be that minimum amount necessary to render the free phosphorus ligand and cobalt-phosphorus ligand complex catalyst that are employed, soluble in the oxo reaction medium. In general, it is considered preferable to employ an excess of that minimum required, although no added benefit is seen in employing a huge excess amount. Accordingly when employed, either as individual compounds or as mixtures, the polar organic solubilizing agents may be present in the oxo reaction medium of this invention in an amount ranging from about 1 to about 60 weight percent of the non-aqueous hydroformylation reaction product composition, amounts of from about 1 to about 35 weight percent being preferred.

Among the preferred polar organic solubilizing agents are amides, sulfoxides, sulfones, and mixtures thereof, the more preferred polar organic solubilizing agents being amides, for instance, N-methyl pyrrolidone.

The improved oxo reaction process of this invention is carried out in the presence of a cobalt-monosulfonated tertiary metal salt phosphine ligand complex catalyst and a free monosulfonated tertiary phosphine metal salt ligand. By "free ligand" is meant monosulfonated tertiary phosphine metal salt ligand that is not complexed with (tied to or bound to) the cobalt atom of the complex.

As noted above the phosphorus ligands employed in this invention as both the phosphorus ligand of the cobalt-phosphorus complex catalyst and free phosphorus ligand are monosulfonated tertiary phosphine metal salt ligands having the general formula (I) depicted above.

Illustrative radicals represented by the $R_1$ and $R_2$ groups in the above monosulfonated tertiary phosphine metal salt ligand formula (I) include monovalent hydrocarbon radicals containing from 1 to 30 carbon atoms, e.g., alkyl radicals including linear or branched primary, secondary or tertiary alkyl radicals, such as methyl, ethyl, n-propyl, isopropyl, butyl, sec-butyl, t-butyl, t-butylethyl, t-butylpropyl, n-hexyl, amyl, sec-amyl, t-amyl, 2-ethylhexyl, n-octyl, iso-octyl, decyl, dodecyl, octadecyl, eicosyl and the like; and cycloalkyl radicals such as cyclopentyl, cyclohexyl, cyclooctyl, cyclohexylethyl, and the like. Moreover, such monovalent hydrocarbon radicals may be substituted if desired with any substitutent that does not unduly adversely effect the desired results of this invention.

Preferably the monovalent hydrocarbon radicals represented by $R_1$ and $R_2$ are selected from the group consisting of alkyl radicals having from $C_1$ to $C_{20}$ carbon atoms, and cycloalkyl radicals having from $C_5$ to $C_{12}$ carbon atoms. More preferably the $R_1$ and $R_2$ groups are each individually an alkyl radical or a cyclohexyl radical. Most preferably the $R_1$ and $R_2$ radicals in a given monosulfonated tertiary phosphine metal salt each individually represent an alkyl radical having from 1 to 10 carbon atoms. Moreover the divalent alkylene radical in said above formula (I) preferably contains from 2 to 5 carbon atoms the more preferred divalent alkylene radicals being 1,3-propylene or 1,4-butylene.

As noted, M in the monosulfonated tertiary phosphine metal salt ligand formula (I) above, represents a metal cation selected from the group consisting of alkali and alkaline earth metals. Illustrative alkali metals include lithium ($Li^+$), sodium ($Na^+$), potassium ($K^+$), cesium ($Cs^+$) and rubidium ($Rb^+$), while illustrative

alkaline earth metals include calcium ($Ca^{++}$), barium ($Ba^{++}$), magnesium ($Mg^{++}$) and strontium ($S^{++}$). Moreover as noted above by the definition of n, the metal salt ligand may contain one or two monosulfonated tertiary phosphine anion molecules corresponding to the positive valence of the metal cation M. preferably n is 1 and M is sodium.

Illustrative preferred monosulfonated tertiary phosphine metal salt ligands include e.g., those having the following general formulas (wherein

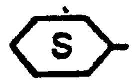

represents a cyclohexyl radical).

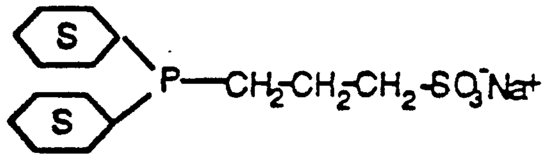

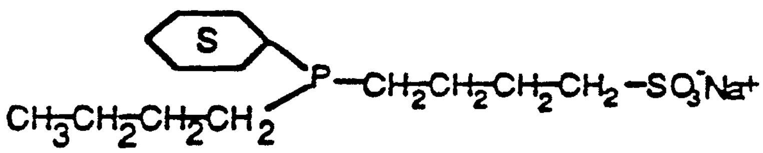

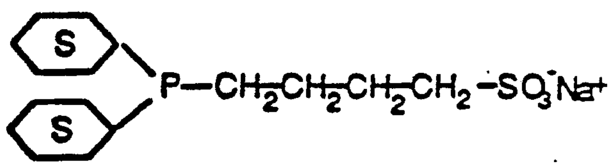

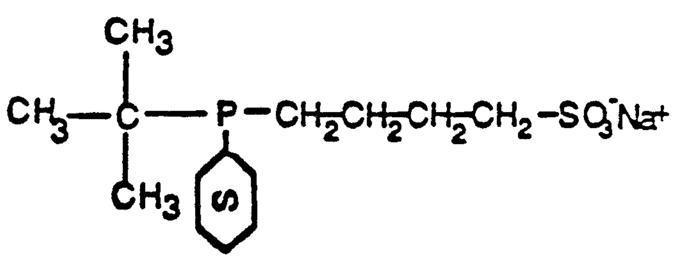

$(CH_3)_2\text{-}P\text{-}C_3H_6\text{-}SO_3^-Na^+$
$(C_2H_5)_2\text{-}P\text{-}C_3H_6\text{-}SO_3^-Na^+$
$(n\text{-}C_4H_9)_2\text{-}P\text{-}C_3H_6\text{-}SO_3^-Rb^+$
$(n\text{-}C_6H_{13})_2\text{-}P\text{-}C_3H_6\text{-}SO_3^-Na^+$
$(n\text{-}C_{10}H_{21})_2\text{-}P\text{-}C_4H_8\text{-}SO_3^-Cs^+$
$[(t\text{-}C_4H_9)_2\text{-}P\text{-}C_4H_8\text{-}SO_3^-]_2Ba^{++}$
$[(n\text{-}C_3H_7)_2\text{-}P\text{-}C_3H_6\text{-}SO_3^-]_2Ca^{++}$
$[(C_2H_5)_2\text{-}P\text{-}C_3H_6\text{-}SO_3^-]_2Mg^{++}$

$[(n\text{-}C_6H_{13})_2\text{-}P\text{-}C_3H_6\text{-}SO_3^-]_2Sr^{++}$
$(iso\text{-}C_3H_7)_2\text{-}P\text{-}C_4H_8\text{-}SO_3^-K^+$
$(iso\text{-}C_3H_7)_2\text{-}P\text{-}C_4H_8\text{-}SO_3^-Na^+$
$(iso\text{-}C_3H_7)_2\text{-}P\text{-}C_3H_6\text{-}SO_3^-Li^+$
$(iso\text{-}C_3H_7)_2\text{-}P\text{-}C_3H_6\text{-}SO_3^-Na^+$
$(t\text{-}C_4H_9)_2\text{-}P\text{-}C_3H_6\text{-}SO_3^-K^+$
and the like.

Such types of monosulfonated tertiary phosphine metal salt ligands employable in this invention and/or methods for their manufacture are known or obvious. For instance such ligands may be prepared e.g., by conventional nucleophilic substitution type reactions such as taught in "Organic Phosphorus Compounds" Vol I, by G. M. Kosolapoff and L. Maier, pp. 41-42 (1972), Wiley-Interscience, for instance by reacting an alkali phosphide, e.g., $R_1R_2PLi$, with a monosulfonated alkyl halide metal salt, e.g., $Cl(CH_2)_xSO_3Li$, to produce the corresponding monosulfonated tertiary phosphine metal salt ligand, e.g., $R_1R_2P(CH_2)_xSO_3Li$, wherein $R_1$ and $R_2$ are the same as defined above and x has a value of 2 to 12. More preferably ligands wherein the divalent alkylene radical is 1,3-propylene or 1,4-butylene radical may be prepared by reacting an alkali phosphide. e.g., $R_1R_2PLi$, with a sultone e.g., $\overline{CH_2(CH_2)_yCH_2SO_2\,O}$ such as gamma-sultone or delta-sultone to produce the corresponding monosulfonated tertiary phosphine metal salt ligand, e.g., $R_1R_2PCH_2(CH_2)_yCH_2SO_3Li$, wherein $R_1$ and $R_2$ are the same as defined above and y has a value of 1 or 2, as shown by the analogous phosphine-sultone reaction disclosed on page 31 of the above mentioned "Organic Phosphorus Compounds" textbook.

As noted above the oxo reaction process of this invention is carried out in the presence of a cobalt-monosulfonated tertiary phosphine metal salt ligand complex catalyst. However, it is to be noted that the successful practice of this invention does not depend and is not predicated on the exact structure of the active cobalt-phosphorus complex species present in the oxo reaction medium of such oxo reaction processes. Such species may be present in their mononuclear, dinuclear and or higher nuclearity forms. Indeed the exact structure is not known. Although it is not intended herein to be bound to any theory or mechanistic discourse, as in the case of heretofore conventional catalytically active cobalt-phosphorus complex species it is believed that the corresponding complex species in the oxo reaction medium of the oxo reaction processes of this invention may in its simplest form consist essentially of cobalt in complex combination with carbon monoxide and monosulfonated tertiary phosphine metal salt ligand. The ultimate composition of the cobalt phosphorus complex may also contain an additional organic ligand or anion i.e. ligand satisfying the coordination sites or nuclear charge of the cobalt metal as in the case of heretofore conventional oxo reaction cobalt-organophosphine catalysts such as e.g., hydrogen and the like. For instance, active oxo reaction catalyst species are generally considered to also contain hydrogen directly bonded to the cobalt particularly in view of the hydrogen gas employed in the oxo reaction process. It is likewise considered that the active species of the cobalt-phosphorus complex present in the oxo reaction mediums of this invention may also be complexed with hydrogen in addition to the monosulfonated tertiary phosphine metal salt and carbon monoxide ligands. Of course, it is also possible that cobalt-phosphorus complex species present in the oxo reaction product composition to be treated in accordance with this invention may be free of such complexed hydrogen as a result of having been removed from the oxo reaction zone of the oxo reactor.

Further it is to be understood that the cobalt-phosphorus complex present in the oxo reaction product compositions produced by the oxo reaction process of this invention can be any such complex mixture resulting from the corresponding cobalt-phosphorus complex catalyst present in the oxo reaction medium of the oxo reaction process that produced the particular oxo reaction product composition that is to be treated in accordance with the process of this invention. Such cobalt-phosphorus complex catalysts may be formed by methods known in the art, for instance preformed cobalt (hydrido) carbonyl monosulfonated tertiary phosphine metal salt ligand complex catalysts may be prepared and introduced with the solubilizing agent if necessary into the reaction medium of the oxo reaction process. More commonly the cobalt-monosulfonated tertiary phosphine metal salt ligand complex catalysts of the oxo reaction process are derived from a metal catalyst precursor, such as $Co_2(CO)_8$, and the like, which may be introduced along with the monosulfonated tertiary phosphine metal salt ligand and an added organic solubilizing agent, as defined herein, if necessary for the in situ formation of the active oxo reaction catalyst. In any event, it is sufficient for the purpose of this invention to understand that carbon monoxide, hydrogen and monosulfonated tertiary phosphine metal salt are all ligands that are capable of being complexed with the cobalt metal and that a cobalt-monosulfonated tertiary phosphine metal salt ligand complex catalyst is present in the oxo reaction medium of the oxo reaction process of this invention and a resultant cobalt-monosulfonated tertiary phosphine metal salt ligand complex is present in the oxo reaction product compositions produced and

treated by the process of this invention.

The amount of such cobalt-phosphorus complex catalyst present in the oxo reaction medium need only be that minimum amount necessary to provide the cobalt metal concentration desired to be employed and which will furnish the basis for at least that catalytic amount of cobalt necessary to catalyze the oxo reaction process. In general, the amount of cobalt-phosphorus complex catalyst present in the oxo reaction medium of a given oxo reaction process may be from about 0.1 to about 2.0 percent by weight of said complex catalyst, calculated as cobalt metal, based on the amount of olefinic compound employed. Preferably amounts ranging from about 0.2 to 1.0 percent by weight of the complex catalyst should be sufficient foz most oxo reaction processes.

As noted above the monosulfonated tertiary phosphine metal salt ligands defined herein are employed in this invention as both the phosphorus ligand of the cobalt-phosphorus ligand complex catalyst, as well as, the free phosphorus ligand that is also present in the oxo reaction medium of the oxo reaction process of this invention. In addition, it is to be understood that while the phosphorus ligand of the cobalt-monosulfonated tertiary phosphine metal salt ligand complex catalyst and free monosulfonated tertiary phosphine metal salt ligand present in the oxo reaction medium of a given oxo reaction process and its corresponding oxo reaction product composition are normally the same, different monosulfonated tertiary phosphine metal salt ligands, as well as, mixtures of two or more different monosulfonated tertiary phosphine metal salt ligands may be employed for each individual purpose, if desired. The oxo reaction process may be carried out in any excess amount of free ligand desired, e.g., at least one mole of free monosulfonated tertiary phosphine metal salt ligand per mole of cobalt present in the oxo reaction medium. In general amounts of free phosphorus ligand of from about 1 to about 10, and preferably from about 1 to about 5 moles per mole of cobalt metal present in the oxo reaction medium should be suitable for most oxo reaction processes.

Accordingly the oxo reaction of this invention may be readily carried out by forming an oxo reaction medium comprising the components defined herein, such as an olefinic compound, carbon monoxide and hydrogen gases, a solubilized cobalt-monosulfonated tertiary phosphine metal salt ligand complex catalyst, solubilized free monosulfonated tertiary phosphine metal salt ligand, and a suitable polar organic solvent for said complex catalyst and said free ligand, in any suitable conventional oxo reactor, such as e.g., a vertical high-pressure tube, in the desired proportions, and effecting the oxo reaction under the conditions of temperature and pressure defined herein to produce the desired oxo reaction product composition of alcohols and aldehydes. It is of course to be understood that the oxo reaction of the oxo process of this invention is a non-aqueous reaction, i.e., conducted in the absence or essential absence of water, which is to say that any water, if present at all in the oxo reaction medium, is not present in an amount sufficient to cause either the oxo reaction or said medium to be considered as encompassing a separate aqueous or water phase or layer in addition to an organic phase. Similarly the oxo reaction product composition is likewise so non-aqueous (prior of course to any deliberate addition of water as called for by the subject invention). Moreover, it is to be further understood that the conditions and factors of the oxo reaction of this invention which will result in a desired oxo reaction product composition having a ratio of alcohols to aldehydes of at least 3 to 1 should be well within the ability of one skilled in the art and easily obtainable by following the teachings of this invention and routine experimentation.

Such alcohol and aldehyde products will of course correspond to those alcohols and aldehydes derived from the particular olefinic compound starting material and may contain from $C_7$ to $C_{31}$ carbon atoms, e.g., one more carbon atom than the starting olefinic compound. The oxo reaction products are normally produced as mixtures of both alcohols and aldehydes and such compound products are themselves, often further mixtures of their straight-chained and branched-chain species. Accordingly the preferred alcohol and aldehyde products are alcohols and aldehydes containing from 7 to 17 carbon atoms, derived from olefins containing from 6 to 16 carbon atoms, and mixtures thereof. Preferably the oxo reaction process of this invention is carried out in such a manner that alcohol is the predominate oxo reaction product. As noted oxo reaction products compositions in which at least 75 percent thereof are alcohols and no more than 25 percent thereof are aldehydes are preferred, while oxo reaction products in which at least 90 percent thereof are alcohols and no more than 10 percent thereof are aldehydes are particularly preferred.

Oxo reaction product compositions obtained from said oxo reaction processes may then be treated in accordance with this invention to separate and recover the alcohol and aldehyde products contained therein by treating (mixing) said oxo reaction product compositions with added water or both added water and an added non-polar hydrocarbon compound and by phase separation forming a non-polar phase consisting essentially of the alcohol and aldehyde oxo products and whatever other non-polar compounds, e.g., added non-polar compound if employed, that might be present in the treated composition, and a polar phase consisting essentially of the cobalt-monosulfonated tertiary phosphine metal salt ligand complex, the free

monosulfonated tertiary phosphine metal salt ligand, the polar organic solubilizing agent for said complex and said free ligand, and the added water employed in said treatment and whatever other polar compounds that might be present in the treated composition.

Such oxo reaction product compositions may be readily obtained, e.g., by removal of all or some of the oxo reaction medium from the oxo reactor vessel and such can be accomplished in any conventional manner, e.g., in a continuous oxo process, a portion of the ligand oxo reaction medium that has already produced such alcohol and aldehyde products may be merely continuously pumped from the reactor. Of course the subject invention could also be conducted in a batch type process manner if desired.

All or part of the corresponding oxo reaction product composition so obtained may then be treated with the added water or both added water and an added non-polar hydrocarbon compound in accordance with the subject invention and the treated composition separated into two distinct liquid layers or phases. Said treatment of the oxo reaction product composition merely involves thoroughly mixing the added water or both added water and added non-polar hydrocarbon compound with said composition and said treatment can be carried out in any conventional manner or fashion. Said treatment does not require any special equipment or mixing device, although obviously a thorough mixing of the liquids is desired and harsh mixing which might cause an emulsion is preferably to be avoided. Accordingly, any conventional, suitable mixing equipment and procedure may be employed, e.g., co-current or counter-current mixing, spray columns, static mixing, etc. For example an advantageous method might be to add the water or water and non-polar hydrocarbon compound to the oxo reaction product composition and mix same in one or more spray columns and pass the treated composition on to any conventional type decanter vessel for settling of the two different (i.e., polar and non-polar) liquid phases that are rapidly formed in said vessel. Said mixing treatment and resulting separation, both of which may be accomplished in a matter of minutes, may be carried out at any suitable liquid temperature and pressure. For example, said mixing and phase separation may be carried out at a pressure of from about 1 to about 1500 psia, although lower or higher pressures could be employed if desired. Preferably said mixing and phase separation may be carried out at from about 1 to about 500 psia and more preferably at about atmospheric pressure. It is also preferred to avoid temperatures that are so low that the water employed might freeze (bearing in mind that the organic polar solubilizing agent may lower the freezing point of the water) or that are higher than the boiling points of the alcohols involved. Accordingly, said mixing and resulting phase separation may be carried out at a liquid temperature of from about -10 °C to 150 °C, although it may be preferred to carry out said mixing and phase separation at a liquid temperature of from about 20 °C. to about 130 °C and more preferably from about 20 °C. to about 110 °C. Cooling the oxo reaction product composition to a liquid temperature of from about 20 °C. to 60 °C. prior to or during said mixing treatment and/or phase separation may aid in obtaining a more complete phase separation of the non-polar and polar compounds involved.

It is to be further understood that while the subject invention is preferably directed to treating a oxo reaction product composition that has been directly obtained by removal of a corresponding liquid oxo reaction medium from the oxo reactor, the oxo reaction product compositions to be treated in accordance with this invention also encompass any subsequent oxo reaction product composition derived from such an initial composition so obtained, provided of course that said subsequently derived composition contains least some amount of each of the four main ingredients defined herein, i.e., the alcohol and aldehyde product, the cobalt-phosphorus ligand complex, the free phosphorus ligand and the polar organic solubilizing agent for said complex and said free ligand. Moreover, it is to be further understood that the amounts of each of said four main ingredients in such subsequently derived oxo reaction product compositions need not necessarily be the same as those amounts of such ingredients present in the oxo reaction product composition from which such subsequent compositions may be derived. Thus, if desired, an initial oxo reaction product composition pay be subjected to any suitable pretreatment procedure in order to arrive at a subsequently derived oxo reaction product composition starting material to be treated in accordance with this invention.

The added nonppolar hydrocarbon compound employable to help effect the phase separation of this invention can be any non-polar hydrocarbon compound containing from $C_6$ to $C_{30}$ carbon atoms. Illustrative non-polar hydrocarbons include, e.g., alkanes containing from $C_6$ to $C_{30}$ carbon atoms be they of straight or branched chain structure, such as hexane, heptane, octane, nonane, decane, undercane, dodecane, tridecane, tetradecane, pentadecane, hexadecane, heptadecane, octadecane, nonadecane, eicosane, docosane, tetracosane, hexacosane, octacosane, triacontane and the like; olefinic compounds containing from $C_6$ to $C_{30}$ carbon atoms, such as those corresponding to the olefinic compounds employed in the oxo reaction process from which the oxo reaction product compositions may be derived, e.g., those olefinic hydroformylation starting materials discussed herein above, especially alpha-olefins containing from $C_6$ to $C_{30}$ carbon atoms; cycloaliphatic compounds containing from $C_6$ to $C_{12}$ carbon atoms, such as cyclohexane, cyclooctane; and the like. Of course, it is to be understood that such non-polar hydrocarbon compounds may be substituted with any substituent that does not adversely effect the phase separation process of this invention. For example, illustrative substituted alkanes include corresponding flurocarbons, and the like. Moreover, mixtures of two or more different non-polar hydrocarbon compounds can be employed if desired. Conveniently the non-polar hydrocarbon compound, when employed, may be one containing the same number of carbon atoms as the olefinic compound that was employed to produce the desired alcohol and aldehyde product, although such is not considered necessary. Preferably, the non-polar hydrocarbon is a saturated straight chain alkane containing from $C_6$ to $C_{30}$ carbon atoms.

The order of addition of the water and non-polar hydrocarbon compound when employed to the oxo reaction product composition is immaterial and they may be added separately and/or simultaneously, or premixed and then added if desired. Moreover, the amount of added water and amount of added non-polar hydrocarbon when employed is not narrowly critical and need only be that minimum amount sufficient to induce the desired phase separation between the non-polar alcohol and aldehyde products and the polar ingredients of the oxo reaction product composition to be treated. Of course, it is to be understood that the terms "added water" and "added non-polar hydrocarbon" as employed herein refer to water and non-polar hydrocarbons that have been deliberately added to the oxo reaction product

D-15864 composition for the purpose of the phase separation process of this invention, in contrast e.g. to non-polar hydrocarbons that might already be present in said oxo reaction product compositions as an ancillary result of the oxo reaction process itself e.g. unreacted olefin, in situ produced hydrocarbons, hydrocarbons present as the result of employing impure olefin starting materials, and the like, although such amounts of ancillary type non-polar hydrocarbons, if and when present in said oxo reaction product composition may lessen the amount of deliberately added non-polar hydrocarbon necessary to achieve a particular desired result of phase separation. Indeed it may be possible to deliberately added or provide for some or all of the non-polar hydrocarbon to be present in the oxo reaction medium containing the alcohol and aldehyde product thereby rendering it necessary to add only water or water and a lesser amount of added non-polar hydrocarbon to the oxo reaction product composition to achieve the desired phase separation benefit that occurs when both added water and added non-polar hydrocarbon are employed. Thus it is to be further understood that while optimization of the amount of added water or sum amount of added water and added non-polar hydrocarbon employed necessary to achieve the best results and efficiency desired in a given situation. will be dependent upon one's experience in the utilization of the subject phase separation procedure, such should be easily obtainable by following the teachings of this invention and simple routine experimentation.

Accordingly, the subject phase separation procedure of this invention comprises mixing the oxo reaction product composition with from about 2 to about 60 percent by weight, more preferably from about 2 to about 3 0 percent by weight of added water, and from 0 to about 60 percent by weight, more preferably from about 2 to 30 percent by weight of an added non-polar hydrocarbon compound, said amounts of added water and added non-polar hydrocarbon compound being based on the total weight of said oxo reaction product composition starting material, and by phase separation forming a liquid non-polar phase consisting essentially of alcohols and aldehydes and the added non-polar hydrocarbon compound when employed, and a liquid polar phase consisting essentially of the added water, the cobalt phosphorus complex, the free phosphorus ligand and an organic solubilizing agent for said complex and said free ligand. Preferably the amount of added water employed or the sum amount of added water and added non-polar hydrocarbon employed is at least sufficient to provide phase separation of at least about 70 weight percent and more preferably at least about 90 weight percent of the alcohols and aldehydes contained in said oxo reaction product composition from at least about 95 weight percent and more preferable at least about 98 weight percent of the cobalt-phosphorus ligand complex calculated as cobalt metal. More preferably said amount of added water employed or the said sum amount of added water and added non-polar hydrocarbon employed is at least sufficient to also provide said phase separation of said alcohol and aldehyde from at least about 95 weight percent and more preferably at least about 98 weight percent of the free phosphorus ligand also contained in said oxo reaction product composition, in addition to providing said alcohol and aldehyde phase separation from said cobalt-phosphorus ligand complex. Most preferably said amount of added water employed or said sum amount of added water and added non-polar hydrocarbon employed is at least sufficient to further also provide said phase separation of said alcohol and aldehyde from at least 75 weight percent and more preferably at least 85 weight percent of the polar organic solubilizing agent for said complex and said free ligand also contained in said oxo reaction product composition, in addition to providing said alcohol and aldehyde phase separation from said cobalt-phosphorus ligand complex and said free phosphorus ligand.

When employing both added water and an added non-polar hydrocarbon compound, instead of merely

water alone, the sum amount of both said additions may preferably be in the range of from about 5 to about 60 percent by weight, and more preferably about 10 to about 50 percent by weight, based on the total weight of the non-aqueous oxo reaction product composition to be treated. It is thought that the employment of both added water and an added non-polar hydrocarbon may result in a beneficial synergistic effect with regard to better achieving the optimum overall desired phase separation results discussed above with regard to all four of the main components in the oxo reaction product composition, as compared to that achievable when adding comparative amounts of only water alone or only a non-polar hydrocarbon compound alone.

In general, it is preferred to treat the oxo reaction product composition by mixing it with added water or added water and an added non-polar hydrocarbon compound in any suitable manner and allowing the treated composition to settle into a non-polar and polar phase in any conventional liquid decanter and separate and recover said non-polar phase by any conventional method such as by simple decantation or pumping. Alternatively, if desired, the subject phase separation may be carried out directly in any conventional cocurrent or counter-current liquid-liquid extractor.

The alcohol and aldehyde containing liquid crude non-polar phase obtained by the subject invention may be further purified from non-polar hydrocarbon compounds and/or possible polar compounds that might also be present in said non-polar phase, if desired and such can be accomplished by any conventional means. For example, polar compounds may be removed from the alcohol and aldehyde containing liquid crude non-polar phase so obtained by employing any conventional cocurrent or counter-current, liquid-liquid extractor, while separation of the alcohol and aldehyde products from each other and from other non-polar compounds may be accomplished by distillation. For instance, it may be preferred that the alcohol and aldehyde containing liquid crude non-polar phase obtained, for economic reasons at least be purified from the possible amounts of polar compounds that may be present therein, such as the cobalt-phosphorus ligand complex, free phosphorus ligand, and the polar organic solubilizing agent for said complex and said free ligand, by employing a counter-current, liquid-liquid extractor. For example, water may be used in a counter-current, liquid-liquid extractor to remove the polar compounds from the alcohol and aldehyde containing liquid crude non-polar phase obtained by this invention. Moreover, if desired a small amount of corresponding monosulfonated tertiary phosphine metal salt ligand, such as present in the oxo reaction product compositions or any other suitable rhodium scavenger, may be added along with the water to the extractor to help scavenge any cobalt that might be present. The aqueous containing polar composition obtained from the extractor may be recycled into the system as desired. For instance, if desired, all or a portion of the aqueous polar solution recovered from the extractor may be recycled and employed as the source of water added to the oxo reaction product composition to be treated in accordance with this invention.

The aqueous cobalt-phosphorus complex containing liquid polar phase that has been phase separated from the alcohol and aldehyde product by the phase separation procedure of the subject invention, after removal of the water, may if desired, be recycled back to the oxo reactor of the oxo reaction process in order to achieve a continuous, liquid catalyst recycle, oxo reaction process. The water may be removed from the aqueous cobalt-phosphorus compjex containing polar phase by any conventional method, such as by pumping it to any conventional vaporizer-separator. The non-aqueous cobalt phosphorus complex containing polar composition (which also contains free phosphorus ligand and polar organic solubilizing agent for said complex and said free ligand) accumulated from the vaporizer-separator may then be recycled back to the oxo reactor. Of course if desired two or more vaporizer-separators can be employed so that the aqueous separation process is repeated, e.g., the aqueous liquid removed from the first vaporizer being employed as the feed for the second vaporizer-separator and the vaporizer overhead accumulated from the second vaporizer recycled to the first vaporizer-separator.

Removal of the water from an aqueous cobalt-phosphorus ligand complex containing polar phase by means of a vaporizer-separator as discussed above, may and normally will also cause some vaporization removal of the polar organic solubilizing agent also present in the aqueous polar phase. Thus, while the water and other vaporized materials obtained from a vaporizer-separator may be condensed and reused if desired, e.g., as part of the water added to the liquid-liquid extractor discussed above, economic reasons may dictate the desirability of first separating and recovering the vaporized polar organic solubilizing agent from the water and reusing the condensed polar non-aqueous organic solubilizing agent composition so obtained by recycling it back to the oxo reactor. Likewise, it may be desirable to also separate and recover organic polar solubilizing agent from the water of the aqueous solution obtained from a liquid-liquid extractor as discussed above. Such separation of the water and organic polar solubilizing agent may be accomplished by any conventional method, such as by employing a conventional distillation column wherein said materials are refluxed to separate and obtain both water and the organic polar solubilizing agent composition from the distillation column. The separated non-aqueous organic polar solubilizing agent stream so recovered may then be recycled to the oxo reactor in any manner desired, while the separated water so obtained may be recycled and reused in any manner desired, e.g., as part or all of the water added to the liquid-liquid extractor.

Accordingly, the subject invention involving the phase separation of the alcohol and aldehyde product from reaction product composition not only provides a unique technical advancement in the art with regard to furnishing a method which may allow for improved overall separation of the alcohol and aldehyde product, from all three other main components of the oxo reaction product composition, i.e., the cobalt complex, the free ligand and the polar organic solvent for said complex and said free ligand, it also provides for a uniquely self-contained phase separation system, wherein, e.g., the small amount of added water employed need only be added to the system a single time, since the same water employed to effect said separation may be continuously recycled and reused, in a closed system. There is no need for additional charges of supplemental new water to such a closed system although additional amounts of supplemental new water could be employed if desired. Further an additional benefit of this invention is the resultant improved overall continuous liquid catalyst recycle, oxo reaction process that is also provided for by the subject invention along with its attendant benefits as disclosed herein.

The alcohols and aldehydes obtained by the process of this invention have any number of well known valuable uses, e.g., they can be used in he production of detergents as well as solvents and plasticizers for polymers. Moreover such mixtures of alcohol and aldehyde products can be readily and easily separated by any conventional manner, if desired, e.g., by distillation.

The following example is illustrative of the present invention and is not to be regarded as limitative. It is to be understood that all parts, percentages and proportions referred to herein and in the appended claims are by weight unless otherwise indicated.

## EXAMPLE 1

The corresponding oxo reaction alcohol and aldehyde product (i.e., tridecanols and tridecanals) of dodecene-1 may be produced by forming an oxo reaction medium in a suitable reaction vessel, said medium containing dodecene-1, a cobalt catalyst precursor composition of $Co_2(CO)_8$ and a monosulfonated tertiary phosphine metal salt ligand having the formula:

$(iso\text{-}C_3H_7)_2\text{-}P\text{-}C_3H_6SO_3^{-}Na^{+}$

and N-methyl pyrrolidone ss the polar organic solvent for t he complex catalyst and free ligand, and reacting said dodecene-1 with hydrogen and carbon monoxide gas at a reaction temperature of about 190°C to about 195°C and a pressure of about 1000 psig. until the desired tridecanol-tridecanal reaction product mixture has been formed.

The alcohol and aldehyde oxo reaction product composition obtained from the above described oxo reaction process may then be treated (mixed) at ambient temperature (e.g., about 25°C) with added water or with both added water and an added non-polar hydrocarbon compound, e.g. cyclohexane and the treated composition allowed to stand and phase separate into a non-polar phase consisting essentially of the alcohol and aldehyde product (and added non-polar hydrocarbon when used) and a polar phase consisting essentially of cobalt-monosulfonated tertiary phosphine metal salt ligand complex, free monosulfonated tertiary phosphine metal salt ligand, the N-methyl pyrrolidone polar organic solvent for said complex and said free ligand and the added water. Said non-polar and polar phases may then be separated by decantation. The efficiency of said phase separation procedure and the percentages of each compound in each ligand layer may be shown by analyzing both the non-polar phase and the polar phase, e.g., by gas chromatography (GC) for the alcohol, aldehyde, unreacted olefin and polar organic solvent compounds; by high performance liquid chromatography (HPLC) for the free monosulfonated tertiary phosphine metal salt ligand and by atomic absorption spectroscopy (ASS) for cobalt metal.

## EXAMPLE 2

A corresponding oxo reaction alcohol and aldehyde product (i.e., tridecanols and tridecanals) of dodecene-1 was produced by forming an oxo reaction medium in a suitable reaction vessel, said medium containing about 25 mL. of dodecene-1, a cobalt catalyst precursor composition of about 0.683 grams of

$Co_2(CO)_8$ and a about 5.45 grams of the sodium salt of 4 - (dicyclohexylphosphino) - butane sulfonate as the ligand and about 93.9 grams of N-methyl pyrrolidone as the polar organic solvent for the complex catalyst and free ligand, and reacting said dodecene-1 with hydrogen and carbon monoxide gas at a reaction temperature of about 193 °C and a pressure of about 1040 psig. for about three hours after it took about one hour to heat the reaction medium to about 193 °C. In this example very little alcohol and aldehyde product was formed (a $CO + H_2$ pressure drop of only about 20 psig. occurred over said three hours of reaction) for some unknown reason.

## Claims

1. A process for the production of alcohols and aldehydes by an oxo process comprising reacting an olefinic compound containing from 6 to 30 carbon atoms with carbon monoxide and hydrogen in the presence of a solublized cobalt-phosphorus ligand complex, solubilized free phosphorus ligand and an organic solvent for said complex catalyst and said free ligand, which process comprises employing, as the phosphorus ligand of said complex catalyst and as said free phosphorus ligand, a monosulfonated tertiary phosphine metal salt ligand having the formula:

$$(I) \qquad \left[ \begin{matrix} R_1 \\ R_2 \end{matrix} > P - \text{alkylene} - SO_3^- \right]_n \left[ M^{n+} \right]$$

wherein $R_1$ and $R_2$ each individually represent a radical containing from 1 to 30 carbon atoms selected from the class consisting of alkyl and cycloalkyl radicals, wherein the divalent alkylene radical contains from 2 to 12 carbon atoms, wherein M represents a metal cation selected from the group consisting of alkali and alkaline earth metals, and wherein n has a value of 1 or 2 corresponding to the valance of the particular metal cation represented by M; and employing as the organic solvent for said complex catalyst and said free ligand a polar organic solvent; and (1) phase separating the alcohol and aldehyde products from the oxo reaction product composition of said process by mixing said composition with from about 2 to about 60 percent by weight of added water and from 0 to about 60 percent by weight of an added non-polar hydrocarbon, said amounts of added water and added non-polar hydrocarbon compound being based on the total weight of said composition, to form an alcohol and aldehyde product containing non-polar phase and a cobalt-monosulfonated tertiary phosphine metal salt ligand complex containing polar phase, and (2) recovering said non-polar phase from said polar phase, with the proviso that the amount of added water or sum amount of added water and added non-polar hydrocarbon compound employed is at least sufficient to provide phase separation of at least 70 weight percent of the alcohols and aldehydes contained in said composition from at least 95 weight percent of the cobalt-phosphorus ligand complex calculated as cobalt metal also contained in said composition.

2. A process as defined in claim 1, wherein the oxo reaction is conducted at a temperature of from about 160 °C to 275 °C and at a total pressure of carbon monoxide and hydrogen of from 600 psig. to about 6000 psig.

3. A process as defined in Claim 1 or 2, wherein the polar organic solvent is an organic liquid having molecular weight of less than 250 and a Hildebrand solubility value of at least 10, or mixtures of such liquids.

4. A process as defined in Claim 3, wherein the polar organic solvent is selected from the group consisting of amides, sulfoxides, sulfones, and mixtures thereof.

5. A process as defined in any one of Claims 1 to 4, wherein the amount of added water employed or the sum amount of added water and added non-polar hydrocarbon compound employed and referred to in the proviso clause is at least sufficient to provide phase separation of at least about 70 weight percent of the aldehyde contained in said composition from at least about 95 weight percent of the cobalt-phosphorous complex calculated as cobalt metal and at least about 95 weight percent of the free phosphorous ligand also contained in said composition.

6. A process as defined in any one of Claims 1 to 4, wherein the amount of added water employed or the sum amount of added water and added non-polar hydrocarbon compound employed and referred to in the proviso clause is at least sufficient to provide phase separation of at least about 70 weight percent of the aldehyde contained in said composition from at least about 95 weight percent of the cobalt-phosphorous

complex calculated as cobalt metal, at least about 95 weight percent of the free phosphorous ligand and at least 75 weight percent of the polar organic solvent for said complex and said free ligand also contained in said composition.

7. A process as defined in any one of Claims 1 to 6, wherein $R_1$ and $R_2$ each individually represent an alkyl radical having from 1 to 10 carbon atoms or cyclohexyl radical and wherein $R_3$ is a divalent 1,3-propylene or 1,4-butylene radical.

8. A process as defined in Claim 7, wherein $R_1$ and $R_2$ are both alkyl radicals, wherein n is 1 and M is an alkali metal.

9. A process as defined in any one of Claims 1 to 8, wherein the organic solubilizing agent is N-methyl pyrrolidone.

10. A process as defined in any one of Claims 1 to 9, wherein the added non-polar hydrocarbon compound is a saturated straight chain alkane containing from 6 to 30 carbon atoms.

11. A process as defined in any one of Claims 1 to 10, wherein step (1) consists essentially of mixing said composition with only added water.

12. A process as defined in Claim 11, wherein the amount of added water empolyed in said mixing step is from about 2 to about 30 percent by weight based on the total weight of said composition.

13. A process as defined in any one of Claims 1 to 12, wherein step (1) consists essentially of mixing said composition with both added water and an added non-polar hydrocarbon compound.

14. A process as defined in Claim 13, wherein the amount of added water empolyed in said mixing step is from about 2 to 30 percent by weight and the amount of added non-polar hydrocarbon compound employed in said mixing step is from about 2 to 30 percent by weight, said amounts of added water and added non-polar hydrocarbon compound being based on the total weight of said composition.

15. A process as defined in any one of Claims 1 to 14, wherein the liquid polar phase separated from the liquid non-polar phase is recycled to the oxo reactor of the oxo process after the removal of the water contained in said liquid polar phase.

European Patent Office

# EUROPEAN SEARCH REPORT

Application number: EP 89112856.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 731 486 (ANTHONY G. ABATJOGLOU et al.) * Claims 1,38 * | 1,3,4, 9 | C 07 C 45/50<br>C 07 C 29/16<br>C 07 C 27/22 |
| A | EP - A2 - 0 226 988 (RUHRCHEMIE) * Abstract; page 5, line 28 - page 6, end of first paragraph * | 1 | |
| A | US - A - 4 633 021 (RONNIE M. HANES) * Claims 1,5 * | 1,4,9 | |
| A | US - A - 4 390 729 (ALEXIS A. OSWALD) * Abstract * | 1 | |
| A | FR - A - 2 473 504 (RHONE-POULENC) * Claim 1 * | 1 | **TECHNICAL FIELDS SEARCHED (Int. Cl.4)** |
| A | EP - A1 - 0 024 761 (SHELL INTERNATIONALE RESEARCH) * Abstract * | 1 | C 07 C 27/00<br>C 07 C 29/00<br>C 07 C 45/00<br>C 07 C 31/00<br>C 07 C 47/00<br>B 01 J |
| A | GB - A - 2 028 677 (KURARAY CO.) * Abstract * | 1 | |
| D,A | US - A - 3 959 385 (HANS JUERGEN NIENBURG et al.) * Claim 1 * | 1 | |

The present search report has been drawn up for all claims

| Place of search | Date of completion of the search | Examiner |
|---|---|---|
| VIENNA | 12-10-1989 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document